# EUROPEAN PATENT SPECIFICATION

(11) **EP 1 080 738 B1**
(45) Date of publication and mention of the grant of the patent: **22.09.2004**
(21) Application number: 00830413.1
(22) Date of filing: 09.06.2000
(51) Int. Cl.: A61L 31/16

(54) **Angioplasty stent**
Stent für Angioplastie
Stent pour angioplastie

(30) Priority: 05.08.1999 IT TO990693
(43) Date of publication of application: 07.03.2001
(73) Proprietor: SORIN BIOMEDICA CARDIO S.p.A., 13040 Saluggia (Vercelli) (IT)
(72) Inventor: Vallana, Franco, 10123 Torino (IT); Curcio, Maria, 13040 Saluggia (Vercelli) (IT); Rolando, Giovanni, 10034 Chivasso (Torino) (IT)
(74) Representative: Bosotti, Luciano

(56) References cited:
- EP-A- 0 441 516
- EP-A- 0 941 740
- WO-A-95/03356
- WO-A-99/32167
- US-A- 5 769 883
- US-A- 5 885 609

## Description

The present invention relates to angioplasty stents.

This name is intended to indicate in a general way those devices intended for intravascular application (for example inside a blood vessel), usually carried out by catheterization followed by in-situ expansion in order to exert an action of local support to the lumen.

For a general overview of vascular stents the reader may usefully refer to the work "Textbook of Interventional Cardiology", ed. Eric J. Topol, W.B. Saunders Company, 1994, and in particular section IV of vol. II entitled "Coronary stenting".

The subject has also attracted a large number of patent documents, as witness, for example, EP-A-0 806 190, EP-A-0 850 604, EP-A-0 847 766, EP-A-0 857 470, EP-A-0 875 215, EP-A-0 895 759, EP-A-0 895 760.

The clinical use of these devices, which in recent years has grown considerably in popularity, must address the need to ensure an effective impediment to the phenomenon usually known as restenosis. This is a phenomenon (linked to the manifestation of physiological mechanisms that, furthermore, are not yet clearly understood) whereby the stenosis site, after having been reopened by the implant of the stent, gradually tends to close up again, usually owing to gradual tissue growth.

Various solutions have been proposed for dealing with this problem: they essentially involve a local action designed to impede the phenomena that give rise to the restenosis. In particular, a number of solutions have been investigated in which drugs are released locally or radioactive sources are used locally.

These solutions, especially those based on the localized and controlled release of drugs, have themselves to address the problem of how this localization is to be effectively carried out at the stent implant site. In the case of agents designed to be released by the stent it is important to ensure that the majority of the agents are released gradually into the walls of the vessel rather than being washed away relatively rapidly by the flow of blood passing through the lumen of the stent. This is particularly important because clinical experiments show that the mechanisms of reaction of the vessel subjected to the stent implant occur at the level of a reaction by the vessel wall within a period of time typically ranging from 1 to 6 months from implantation. An example of these solutions is given in US-A-5 885 609, EP-A-0 441 516 and EP-A-0 941 740.

It is an object of the present invention to provide a solution capable of improving on previously attempted solutions, from a number of points of view.

In the first place it is wished to be able to achieve a reliable connection, or anchoring, of the active agents to the stent, and in particular a connection that will not be influenced either by the movement of the stent as it dilates, which is characteristic of the implanting operation, or by possible surface treatments (such as deposition of a surface layer of biocompatible carbon-containing material) which may have been applied to the stent.

In the second place, it is wished to be able to produce a collection of components capable of acting in effect as a "releasing machine" that will deliver a controlled release of restenosis-impeding agents, particularly as regards the possibility of precisely controlling the release kinetics, with the further possibility of selectively controlling, in time or in relation to other parameters, the release of diverse agents.

Again, it is wished that the functions specified above be able to be carried out without negative impacts on the other characteristic aspects of the functionality of the stent, e.g. by ensuring that, whether at the time of implantation or later, the lumen of the stent does not become a site for obstructions to the free flow of blood.

According to the present invention this object is achieved with an angioplasty stent having the characteristics specifically set forth in the claims which follow. The invention also relates to the associated kit and to the components that transport the restenosis-impeding agents.

More specifically the present invention relates to a stent having the features set forth in the preamble of claim 1, which is known e.g. from WO-A-99 32 167 and US-A-5 769 883. The invention also relates to components that transport the restenosis-impeding agents according to the preamble of claim 24, which are also known from the above-cited documents.

The invention will now be described, purely by way of non-restrictive example, with reference to the accompanying drawings, in which:
- Figure 1 is a general perspective view, with some parts removed for clarity of illustration, of an angioplasty stent produced according to the invention,
- Figure 2 is a cross section taken on II-II as marked in Figure 1, reproduced on a larger scale, and
- Figures 3 to 7 illustrate in greater detail the characteristics of certain of the elements making up the stent shown in Figure 1.

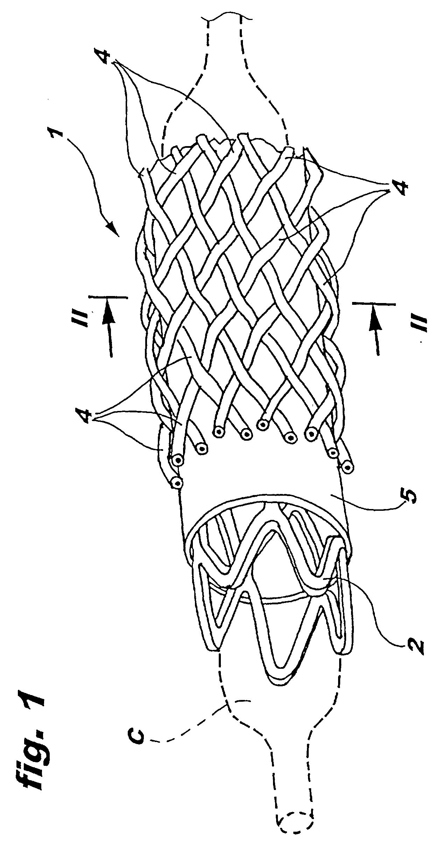

In Figure 1 the numerical reference 1 indicates the whole of a so-called angioplasty stent.

For a general identification of the modes of use and production features of such an implant device, refer to the documentation cited in the introductory part of this description.

By way of an overview, it will be recalled that the stent 1 is usually produced - as a basic structure - in the form of a body whose geometrical envelope is tubular, with a total length usually varying from a few millimetres to some tens of millimetres and a wall thickness (the wall usually having an open structure of links or loops) of the order of, for example, a few hundredths of a millimetre. These features are all with a view to the possible insertion of the stent into a lumen, (such as a blood vessel), in a site suffering from stenosis which is to be corrected. The stent is moved into position, e.g. by catheterization, and than expanded radially from its diameter of insertion, which is of the order of, say, 1 mm, to an expanded diameter of the order of, say, 3-5 mm. In the expanded condition the stent supports the lumen and eliminates the stenosis. The outside diameter when radially contracted is selected so as to allow the stent to be inserted into the lumen where the treatment is being carried out, while the expanded diameter corresponds as a rule to the diameter which it is wished to establish and maintain in the lumen once the stenosis has been eliminated.

Although the principal application of the stent described above refers to the treatment of blood vessels, it is perfectly possible (and therefore included within the scope of the invention) to fit it as a support for any lumen present in the human or animal body.

As far as the methods and principles that enable the stent to be opened (i.e. expanded in situ) are concerned, the solution that is most widely used at present is to employ what is known as a balloon catheter: the stent is mounted around the balloon of the catheter in its contracted condition and the balloon is expanded once the stent has been delivered to the site of the implant (see, for example, Figure 1, where the outline of the catheter balloon, labelled C, has been indicated schematically in broken lines). However, other methods may also be envisaged, such as that of employing superelastic materials which expand the stent once the containment components, designed to keep the stent contracted until it reaches the site of the implant, have been removed. Additionally or alternatively it has also been suggested that the stent be made from materials having a so-called "shape memory" enabling it to be expanded radially in the implant position.

Usually (for more precise indications refer to the bibliographic and patent literature cited in the introduction to the description) the stent is made from a metallic material that reconciles two fundamental requirements for application, that is to say an ability to deform plastically during the expansion and an ability to maintain its expanded form by resisting stresses that would tend to re-close the stent.

These technological issues will not be discussed in detail in the present description inasmuch as they are not in themselves relevant to an understanding of the embodiment of the invention. This is essentially true also for the technology of manufacture of the stent (manufacture from a continuous tubular preform, manufacture from a striplike body which is then closed into a tube, manufacture from a shaped metal wire, etc.).

In order to illustrate the invention, the structure of the stent, which has the general reference 2, can be simply identified with a tubular body capable of being selectively expanded radially by the principles described above. In other words the solution according to the present invention is in fact quite "transparent" or at any rate not appreciably influenced by the specific characteristics of geometry, treatment and technology of manufacture of the basic structure 2 of the stent.

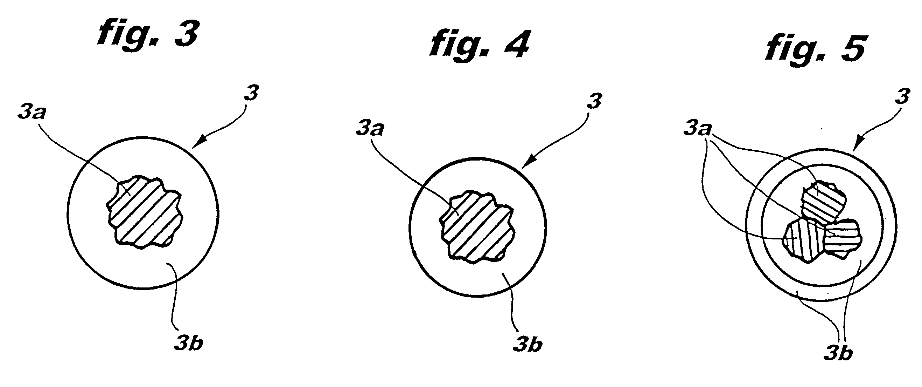

Before turning to a more detailed explanation of the characteristics of the stent 1 it is useful to refer to Figures 3 to 7, which show a number of the components used to make the stent in question.

Specifically, Figures 3 to 5 illustrate, in a number of possible variants, structures 3 of the type commonly known as "nanoparticles".

This name refers in a general way to bodies of spherical or substantially spherical shape having typical diameters of the order of hundreds of nanometres.

The nanoparticles 3 usually comprise a nucleus 3a surrounded by an outer envelope 3b. Figure 5 shows that the nucleus 3a and/or the envelope 3b need not be single-structured but instead multiple-structured (having for example two or more nuclei or subnuclei) and/or stratified, and also if desired with formulations differing from component to component. It is also possible for the nucleus or nuclei. 3a not to be in the approximate centre but instead eccentric relative to the envelope 3b. The rest of this description will however refer for simplicity's sake to nuclei 3a (and 4a) and to envelopes 3b of substantially uniform, homogeneous structure and substantially concentric with each other.

The nucleus 3a consists, as far as is relevant for the purposes of the implementation of the present invention, of any agent capable of exerting an antagonism towards restenosis by the effect of an action of localized release and/or selective penetration into the walls of the vessel where the stent is implanted.

The nucleus 3a may typically consist, for example, of a drug or collection of drugs whose action is anti-inflammatory, antimitotic and/or promotes the processes of repair of the vessel wall such as to mitigate or prevent the reactions on which the process of restenosis is based. However, the particular choice of agent and/or collections of agents is not specifically the subject-matter of the present invention.

The above remarks also apply in substance to the manufacture of the envelope 3b. This last consists, as far as is relevant for the purposes of the implementation of the present invention, of any substance that can be described as "bioerodible". This expression is intended here to denote any substance which, within the context of the present invention (namely implantation in a lumen), tends to be consumed and/or to adopt or demonstrate a porous morphology or any morphology such as to allow the outward diffusion of the substance or substances contained inside the nucleus 3a. The properties of bioerodibility described are typically accompanied by properties of biocompatibility and biodegradability which are important in this specific application.

One possible choice for the production of the envelope 3b is to use substances such as polyethylene glycol (PEG) or polylactic-glycolic acid (PLGA) as the constitutent material. The method of production of these can be taken to be known.

The corresponding release kinetics can be controlled (especially as regards the time required to achieve outward diffusion of the agents contained inside the nucleus 3a) by varying, individually or in combination, parameters such as:
- the composition of the nucleus 3a,
- the composition of the envelope 3b,
- the thickness of the envelope 3b,
- the relative location of the nucleus 3a with respect to the envelope 3b,
- the structure (homogeneous, multiple and/or stratified) of the nucleus 3a, and
- the structure (homogeneous, multiple, stratified and/or porous) of the envelope 3b.

Figures 3 and 4 illustrate, purely by way of example, the option of producing two nanoparticles 3 containing nuclei 3a with dimensions roughly equal to each other in combination with envelopes 3b of differing thicknesses.

In all cases the principles, methods and technologies relating to the production of nanoparticles such as the nanoparticles 3 shown in Figures 3 to 5 should be taken to be well known in the art.

The above remarks also apply in substance to the option of having nanoparticles 3 such as those shown in Figures 3 to 5 to (whether identical to each other or not) accompany fibres 4 that are also preferably made from bioerodible materials such as the materials cited earlier.

The production of the fibres 4 and their provision with the nanoparticles 3 correspond to known solutions. In particular, the production of microfibres in the form of hollow-fibre membranes is well known and well established at the technological level in the manufacture of devices such as dialysers or oxygenators of the blood.

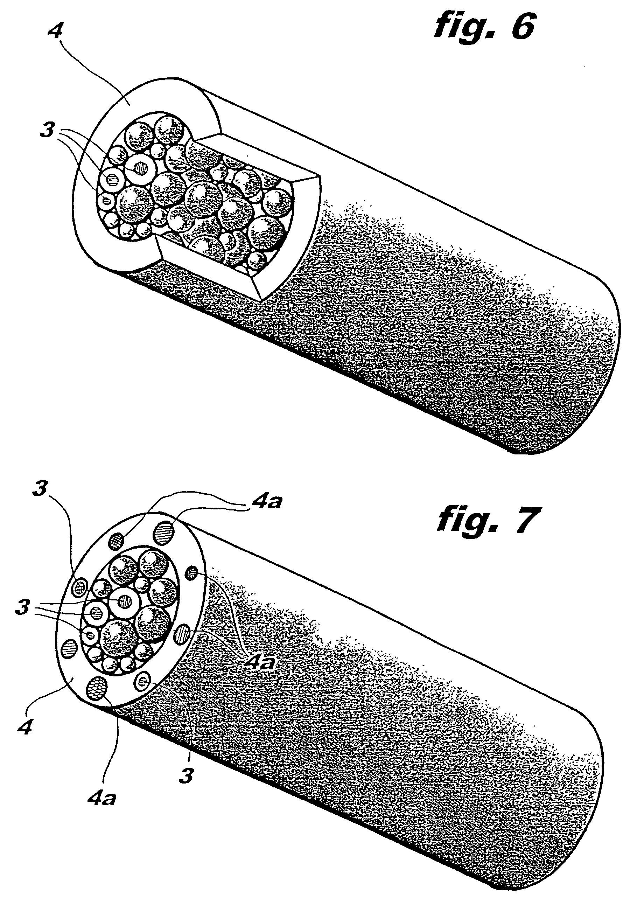

An examination of Figures 6 and 7 shows that a microfibre 4 of the type considered can be used as a container whose interior holds the nanoparticles 3 (Figure 6) and/or as a support (erodible) for the nanoparticles 3 and/or additional nuclei 4a (whether identical to each other or not) substantially equivalent to the nuclei 3a of the nanoparticles 3 as regards their ability to convey restenosis-impeding agents (Figure 7).

Even if not explicitly illustrated in the drawings attached, it is also conceivable, and therefore included in the scope of the present invention, to use, at least locally in the environment of the stent 1, fibres 4 that do not allow for the placing of nanoparticles 3 in the lumen of these fibres 4. In this case, the fact that the fibres 4 have hollow-fibre characteristics is not critical, and the use of fibres 4 of compact structure with no axial cavity is therefore also possible.

By analogy, with reference to the diagram of Figure 6, it is possible to use fibres 4 of a non-bioerodible material that would act exclusively as containers for nanoparticles 3 located inside their lumen and yet allow the diffusion of the agents carried by the nuclei 3a of the nanoparticles 3 if the walls of the fibres are porous.

To give some idea of dimensions, the fibres 4 illustrated in the appended drawings may have diameters of typically between about 30 and 100 µm, with wall thicknesses typically of between 10 and 20 µm, and therefore with corresponding dimensions of the axial cavities typically of between 10 and 60 µm.

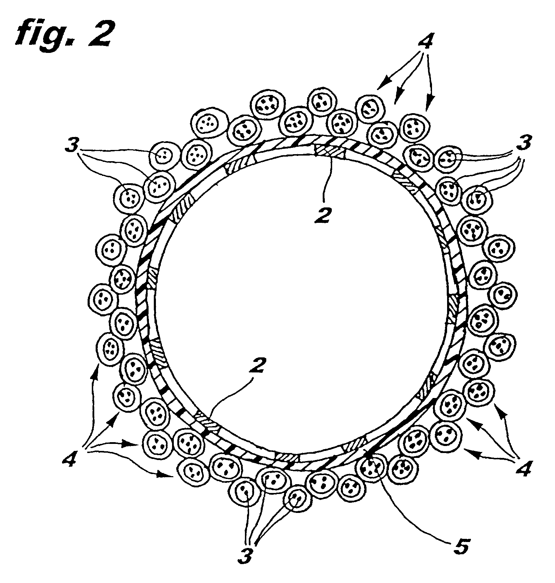

Figure 1 and Figure 2 show how the fibres 4 described above can be used to attach to the base structure 2 of the stent 1 one or more layers of fibres 4, these being optionally interlinked with the structure 2 by exploiting the open configuration of the latter.

The winding arrangement (or more generally the placement) of the fibres 4 on the structure 2 must of course take account of the fact that the fibres 4 are normally laid on the stent 1 when the latter is in its radially contracted position and must not therefore be damaged or altered in their functionality by the radial expansion which typifies the operation of implanting the stent. In other words, the fibres 4 are attached to the structure 2 in such a way as to present virtually no resistance to the dilation characteristic of the implanting of the stent.

This result can be achieved in several different ways.

In the example of an embodiment illustrated here (which, it should be emphasized again, is only an example), the fibres 4 have been wound around the structure of the stent by known processes and devices (see for example US-A-4,952,312) in such a way that the angle formed by the fibres relative to the family of planes perpendicular to the main axis of the stent (in practice the angle of winding of the helical path defined by each fibre) is relatively great, preferably at least 45° and still more preferably greater than 60°.

Alternative solutions are of course possible, such as for example that of making the fibres 4 from elastic materials and/or that of causing the fibres 4 to follow a sinuous path; the purpose being to ensure the fibres can stretch in the longitudinal direction.

For the purpose of laying the fibres 4 on the structure 2 it is also possible to exploit the presence (initially dictated by other reasons explained below) of a sheath or tunic 5 interposed between the structure 2 and the layer or layers of fibres 4 laid around it.

Specifically, it is possible to consider applying the fibres 4 to the structure 2 of the stent 1 by connecting them (e.g. by an adhesive connection) to the outer surface of the sheath 5. In this version it is possible to locate the fibres 4 (whether ordered in a single layer or whether present in two or more layers) so that the fibres 4 run more or less along the generatrices of the imaginary cylindrical surface defined by the structure 2 of the stent. A solution of this kind would ensure that the movement of radial dilation of the structure 2 has no effect on the fibres 4.

The main purpose of the sheath 5 is to prevent unwanted phenomena of prolapse of the fibres 4 into the lumen of the stent 1. These phenomena can occur, for example, when the structure 2 - whether in its entirety or even only partly - has a highly apertured configuration.

Accordingly, the sheath 5 has the function of ensuring that, even following implantation, the fibres 4 preferably stay outside the structure 2 and are interposed between the stent 1 and the vessel wall.

The presence of the sheath 5 is also beneficial at later stages, namely when the agents present in the nuclei 3a and 4a are diffused.

This is because the sheath 5 is able to act at least partly as a barrier and so ensure that the release of the abovementioned active agents takes place preferably and primarily towards the wall of the vessel and not towards the interior lumen of the structure 2, which would mean that the blood would wash them away.

For the manufacture of the sheath 5 it is possible to employ various different materials, preferably with good biocompatibility properties, whether owing to the nature of the constituent material or to the fact that the sheath 5 has been subjected to a treatment such as surface deposition of a layer of biocompatible carbon-containing material. An option which at the moment is thought preferable is to make the sheath 5 from a silicone material. Alternative options are of course possible, such as making the sheath 5 in the form of a tunic of a metallic or polymeric net with a fine mesh capable of reproducing the movement of the stent as it expands.

The option of making the sheath 5 with general characteristics of porosity (or more generally of permeability, which may be more effective on substances moving out through the stent than on substances moving in the opposite direction) is advantageous because it promotes contact between the vessel wall and the blood flowing through the stent, while at the same time preventing the flushing away of the active agents.

The fibres 4 can be laid on the structure 2 at different stages of the process of manufacture and/or preparation for the use of the stent 1.

In particular (irrespective of the principles whereby the fibres 4 are applied to the structure 2) the fibres 4 can be applied in the final stages of manufacture of the stent and thus before the stent is mounted on the catheter C used for its delivery to the site, or at a later stage, for example after the stent 1 has first been mounted on the implanting catheter.

The collection of fibres 4 can therefore constitute a component of the kit (stent + implanting catheter)) which, in the form currently preferred for marketing, constitutes the product made available for the purposes of the implanting operation.

As another alternative it is possible to attach the fibres to the stent structure at an even later stage, that is immediately before proceeding to implant the stent (before or after the stent has been positioned on the implanting catheter). For this reason the collection of fibres 4 may constitute an independent product capable of being marketed, e.g. in different versions included in an assortment and characterized by the presence of different kinds of agent and/or different kinetics of release. In particular this product may take the form of, for example, a small sheet designed to be wound around the stent or may take the form of a tubular sock for fitting onto the stent.

It is clear from the above that the solution described literally provides a "releasing machine" for the controlled release of restenosis-impeding agents.

In particular, the dimensions of the nanoparticles 3 and of the nuclei 3a, 4a are such that even within the limits of a structure of small dimensions such as a stent it is possible to have a very large number of nuclei and consequently to differentiate the natures and properties of the impeding agents within a wide range of choices.

As far as the release kinetics are concerned, it is important to notice that the solution according to the invention makes it possible to act both at the level of the nanoparticles 3 (in the manner indicated earlier) and at the level of the fibres 4, for example by altering the composition, thickness or structure (making it homogeneous, stratified and/or porous) of the fibres 4, as well as on the location of the nuclei within the fibres (locating them directing, as in the case of the nuclei 4a, or indirectly by incorporating them in nanoparticles 3 which in turn are incorporated in the wall of a fibre).

There is also the option of modifying the further parameter represented by the optional porosity of the sheath 5.

Of particular interest is the possibility of operating in a combined way on the abovementioned parameters, by, for example, causing the agents contained in certain nuclei 3a, 4a to diffuse over different time periods to those of the agents contained in other nuclei 3a, 4a in order that the restenosis-impeding action exactly keeps pace with the manifestation of the phenomena that give rise to restenosis itself.

As an example, it may be decided that the release of anti-inflammatory drugs and/or promoters of vessel-wall repair processes should predominate in the acute phases, while antimitotic agents should be released predominantly at a later stage.

Naturally, without affecting the principle of the invention, the details of construction and forms of embodiment can vary widely from those described and illustrated without thereby departing from the scope of the present invention as defined in the annexed claims.

## Claims

1. Stent for angioplasty comprising a structure (2) whose geometrical envelope is essentially tubular and which can be dilated from a radially contracted position to a radially expanded position, wherein the said structure (2) is provided with fibres (4) forming vectors for nuclei (3a, 4a) of restenosis-impeding agents, **characterized in that** the said nuclei (3a, 4a) are at least partly (3a) incorporated in nanoparticles (3) associated with the said fibres (4) and provided with an envelope (3b) of bioerodible material.

2. Stent according to Claim 1, **characterized in that** the said nuclei (3a, 4a) are at least partly contained within the said fibres (4), the said fibres (4) being made of a bioerodible material.

3. Stent according to Claim 2, **characterized in that** the said nuclei (3a, 4a) are at least partly (4a) incorporated in the walls of the said fibres (4).

4. Stent according to Claim 1, **characterized in that** the said fibres (4) are hollow fibres and the said nanoparticles (3) are located inside the cavities of the said hollow fibres (4).

5. Stent according to Claim 1 or Claim 4, **characterized in that** the said nanoparticles (3) are present in different types differentiated by at least one parameter selected from the following group:
- the composition of the nucleus (3a),
- the composition of the envelope (3b),
- the thickness of the envelope (3b),
- the relative location of the nucleus (3a) with respect to the envelope (3b),
- the structure of the nucleus (3a), and
- the structure of the envelope (3b).

6. Stent according to any one of Claims 1 or 4 to 5, **characterized in that** the said nanoparticles (3) have approximate dimensions of between 100 and 200 nanometres.

7. Stent according to any one of the previous claims, **characterized in that** the said fibres (4) are present in different types differentiated by at least one parameter selected from the following group:
- the composition of the fibre (4),
- the thickness of the fibre (4),
- the structure of the fibre (4), and
- the relative location of the said nuclei (3a, 4a) with respect to the fibre (4).

8. Stent according to any one of the previous claims, **characterized in that** the said fibres (4) are, at least in part, solid fibres.

9. Stent according to any one of the previous claims, **characterized in that** the said fibres (4) are attached to the said structure (2) in such a way as to present virtually no resistance to the dilation of the said structure from the said radially contracted position to the said radially expanded position.

10. Stent according to any one of the previous claims, **characterized in that** the said fibres (4) are at least partly interlinked with the said structure (2).

11. Stent according to any one of the previous claims, **characterized in that** the said fibres (4) are longitudinally extensible.

12. Stent according to Claim 9, **characterized in that** the said fibres (4) extend, at least in part, in the general direction of the generatrices of the said essentially tubular structure (2).

13. Stent according to Claim 9, **characterized in that** the said fibres (4) are wound, at least in part, around the said structure in a helical path with a winding pitch of at least 45°.

14. Stent according to Claim 9, **characterized in that** the said fibres (4) are wound, at least in part, around the said structure in a helical path with a winding pitch of at least 60°.

15. Stent according to any one of the previous claims, **characterized in that** the said fibres (4) exhibit, at least in part, a porous structure.

16. Stent according to any one of the previous claims, **characterized in that** a containment sheath or tunic (5) is interposed between the said structure (2) and the said fibres (4).

17. Stent according to Claim 16, **characterized in that** the said sheath (5) is made of a biocompatible material.

18. Stent according to Claim 16 or Claim 17, **characterized in that** the said sheath (5) has an apertured structure.

19. Stent according to any one of Claims 16 to 18, **characterized in that** the said sheath (5) is made of a silicone material.

20. Stent according to any one of Claims 16 to 18, **characterized in that** the said sheath (5) is made of a metallic or polymeric material.

21. Stent according to any one of the previous Claims 16 to 20, **characterized in that** the said sheath (5) is coated with a layer of a biocompatible carbon-containing material.

22. Stent according to Claim 16, **characterized in that** the said fibres (4) are at least partly anchored to the said sheath (5).

23. Implant kit comprising:
- a stent according to any one of the previous claims, and
- a catheter (C) for the in-situ application of the said stent (1).

24. Component with restenosis-impeding action that can be combined with an angioplasty stent comprising a structure (2) whose geometrical envelope is essentially tubular and which can be dilated from a radially contracted position to a radially expanded position, the said component comprises fibres (4) that can be associated to the said structure (2) and that form vectors for nuclei (3a, 4a) of restenosis-impeding agents, **characterized in that** the said nuclei (3a, 4a) are at least partly (3a) incorporated in nanoparticles (3) associated to the said fibres (4) and provided with an envelope (3b) of bioerodible material.

25. Component according to Claim 24, **characterized in that** the said nuclei (3a, 4a) are at least partly contained within the said fibres (4), the said fibres (4) being made of a bioerodible material.

26. Component according to Claim 25, **characterized in that** the said nuclei (3a, 4a) are at least partly (4a) incorporated in the walls of the said fibres (4).

27. Component according to Claim 24, **characterized in that** the said fibres (4) are hollow fibres and the said nanoparticles (3) are located inside the cavities of the said hollow fibres (4).

28. Component according to Claim 24 or Claim 27, **characterized in that** the said nanoparticles (3) are present in different types differentiated by at least one parameter selected from the following group:
- the composition of the nucleus (3a),
- the composition of the envelope (3b),
- the thickness of the envelope (3b),
- the relative location of the nucleus (3a) with respect to the envelope (3b),
- the structure of the nucleus (3a), and
- the structure of the envelope (3b).

29. Component according to any one of Claims 24 or 27 to 28, **characterized in that** the said nanoparticles (3) have dimensions substantially of between 100 and 200 nanometres.

30. Component according to any one of the previous Claims 24 to 29, **characterized in that** the said fibres (4) are present in different types differentiated by at least one parameter selected from the following group:
- the composition of the fibre (4),
- the thickness of the fibre (4),
- the structure of the fibre (4), and
- the relative location of the said nuclei (3a, 4a) with respect to the fibre (4).

31. Component according to any one of the previous Claims 24 to 30, **characterized in that** the said fibres (4) are, at least in part, solid fibres.

32. Component according to any one of the previous Claims 24 to 31, **characterized in that** the said fibres (4) are capable of being associated to the said structure (2) in such a way as to present virtually no resistance to the dilation of the said structure from the said radially contracted position to the said radially expanded position.

33. Component according to any one of the previous Claims 24 to 32, **characterized in that** the said fibres (4) are at least partly interlinkable with the said structure (2).

34. Component according to any one of the previous Claims 24 to 33, **characterized in that** the said fibres (4) are longitudinally extensible.

35. Component according to Claim 32, **characterized in that** the said fibres (4) extend, at least in part, in the general direction of the generatrices of the said essentially tubular structure (2).

36. Component according to Claim 32, **characterized in that** the said fibres (4) are arranged, at least in part, in a helical path with a winding pitch of at least 45°.

37. Component according to Claim 32, **characterized in that** the said fibres (4) are arranged, at least in part, in a helical path with a winding pitch of at least 60°.

38. Component according to any one of the previous Claims 24 to 37, **characterized in that** the said fibres (4) exhibit, at least in part, a porous structure.

39. Component according to any one of the previous Claims 24 to 38, **characterized in that** it comprises a containment sheath or tunic (5) capable of being interposed between the said structure (2) and the said fibres (4).

40. Component according to Claim 39, **characterized in that** the said sheath (5) is made of a biocompatible material.

41. Component according to Claim 39 or Claim 40, **characterized in that** the said sheath (5) has an open structure.

42. Component according to any one of Claims 39 to 41 42, **characterized in that** the said sheath (5) is made of a silicone material.

43. Component according to any one of Claims 39 to 41 , **characterized in that** the said sheath (5) is made of a metallic or polymeric material.

44. Component according to any one of the previous Claims 39 to 43, **characterized in that** the said sheath (5) is coated with a layer of a biocompatible carbon-containing material.

45. Component according to Claim 39, **characterized in that** the said fibres (4) are at least partly anchored to the said sheath (5).

46. Component according to any one of Claims 24 to 45 in the form of a small sheet designed to be wound around the structure of the stent (1).

47. Component according to any one of Claims 24 to 45 , in the form of a tubular sock capable of being fitted onto the structure (2) of the stent (1).

48. Assortment of components according to any one of Claims 24 to 47, comprising a plurality of the said components that are **characterized by** the presence of respective different types of the said restenosis-impeding agent.

49. Assortment of components according to any one of Claims 24 to 47, comprising a plurality of the said components that are **characterized by** respective differentiated kinetics of release of the said restenosis-impeding agent.

## Patentansprüche

1. Stent für Angioplastie, aufweisend einen Aufbau (2), dessen geometrische Umhüllung im Wesentlichen rohrförmig ist und der von einer radial zusammengezogenen zu einer radial expandierten Position aufgeweitet werden kann, wobei der Aufbau (2) mit Fasern (4) versehen ist, welche Träger für Kerne (3a, 4a) von Restenosis verhindernden Mitteln bilden, **dadurch gekennzeichnet, dass** die Kerne (3a, 4a) mindestens teilweise (3a) in Nanoteilchen (3) eingebaut sind, welche den Fasern (4) zugeordnet und mit einer Umhüllung (3b) aus bioerodierbarem Material versehen sind.

2. Stent nach Anspruch 1, **dadurch gekennzeichnet, dass** die Kerne (3a, 4a) mindestens teilweise in den Fasern (4) enthalten sind, wobei die Fasern (4) aus bioerodierbarem Material bestehen.

3. Stent nach Anspruch 2, **dadurch gekennzeichnet, dass** die Kerne (3a, 4a) mindestens teilweise (4a) in den Wänden der Fasern (4) eingebaut sind.

4. Stent nach Anspruch 1, **dadurch gekennzeichnet, dass** die Fasern (4) hohle Fasern sind und dass die Nanoteilchen (3) innerhalb der Hohlräume der hohlen Fasern (4) angeordnet sind.

5. Stent nach Anspruch 1 oder 4, **dadurch gekennzeichnet, dass** die Nanoteilchen (3) in verschiedenen Typen vorliegen, differenziert durch mindestens einen aus der nachfolgenden Gruppe ausgewählten Parameter:
- die Zusammensetzung des Kerns (3a),
- die Zusammensetzung der Umhüllung (3b),
- die Dicke der Umhüllung (3b),
- der relative Ort des Kerns (3a) in Bezug zur Umhüllung (3b),
- der Aufbau des Kerns (3a), und
- der Aufbau der Umhüllung (3b).

6. Stent nach einem der Ansprüche 1 oder 4 bis 5, **dadurch gekennzeichnet, dass** die Nanoteilchen (3) ungefähre Abmessungen zwischen 100 und 200 Nanometern aufweisen.

7. Stent nach einem der vorhergehenden Ansprüche, **dadurch gekennzeichnet, dass** die Fasern (4) in verschiedenen Typen vorliegen, differenziert durch mindestens einen aus der nachfolgenden Gruppe ausgewählten Parameter:
- die Zusammensetzung der Faser (4),
- die Dicke der Faser (4),
- der Aufbau der Faser (4), und
- der relative Ort des Kerns (3a, 4a) in Bezug zu der Faser (4).

8. Stent nach einem der vorhergehenden Ansprüche, **dadurch gekennzeichnet, dass** die Fasern (4) mindestens teilweise feste Fasern sind.

9. Stent nach einem der vorhergehenden Ansprüche, **dadurch gekennzeichnet, dass** die Fasern (4) an dem Aufbau (2) derart befestigt sind, dass sie praktisch keinen Widerstand für die Aufweitung des Aufbaus von der radial zusammengezogenen Position zu der radial expandierten Position darstellen.

10. Stent nach einem der vorhergehenden Ansprüche, **dadurch gekennzeichnet, dass** die Fasern (4) mindestens teilweise mit dem Aufbau (2) verbunden sind.

11. Stent nach einem der vorhergehenden Ansprüche, **dadurch gekennzeichnet, dass** die Fasern (4) in Längsrichtung ausdehnbar sind.

12. Stent nach Anspruch 9, **dadurch gekennzeichnet, dass** die Fasern (4) sich mindestens teilweise in der allgemeinen Richtung der Erzeugenden des im Wesentlichen rohrförmigen Aufbaus (2) erstrecken.

13. Stent nach Anspruch 9, **dadurch gekennzeichnet, dass** die Fasern (4) mindestens teilweise um den Aufbau herum in einem schraubenförmigen Weg mit einer Schraubensteigung von mindestens 45° gewickelt sind.

14. Stent nach Anspruch 9, **dadurch gekennzeichnet, dass** die Fasern (4) mindestens teilweise um den Aufbau herum in einem schraubenförmigen Weg mit einer Schraubensteigung von mindestens 60° gewickelt sind.

15. Stent nach einem der vorhergehenden Ansprüche, **dadurch gekennzeichnet, dass** die Fasern (4) mindestens teilweise einen porösen Aufbau aufweisen.

16. Stent nach einem der vorhergehenden Ansprüche, **dadurch gekennzeichnet, dass** eine Einschließungshülle oder ein Häutchen (5) zwischen den Aufbau (2) und die Fasern (4) eingefügt ist.

17. Stent nach Anspruch 16, **dadurch gekennzeichnet, dass** die Hülle (5) aus bioverträglichem Material besteht.

18. Stent nach Anspruch 16 oder 17, **dadurch gekennzeichnet, dass** die Hülle (5) einen geöffneten Aufbau aufweist.

19. Stent nach einem der Ansprüche 16 bis 18, **dadurch gekennzeichnet, dass** die Hülle (5) aus Silikonmaterial besteht.

20. Stent nach einem der Ansprüche 16 bis 18, **dadurch gekennzeichnet, dass** die Hülle (5) aus einem metallischen oder polymeren Material besteht.

21. Stent nach einem der vorhergehenden Ansprüche 16 bis 20, **dadurch gekennzeichnet, dass** die Hülle (5) mit einer Schicht aus bioverträglichem, Kohlenstoff enthaltendem Material beschichtet ist.

22. Stent nach Anspruch 16, **dadurch gekennzeichnet, dass** die Fasern (4) mindestens teilweise in der Hülle (5) verankert sind.

23. Implantatsatz, aufweisend:
- einen Stent gemäß einem der vorhergehenden Ansprüche, und
- einen Katheter (C) für die in situ Anwendung des Stents (1).

24. Komponente mit Restenosis verhindernder Wirkung, die mit einem Angioplastie-Stent kombiniert werden kann, aufweisend einen Aufbau (2), dessen geometrische Umhüllung im Wesentlichen rohrförmig ist und welcher von einer radial zusammengezogenen Position zu einer radial expandierten Position aufgeweitet werden kann, wobei die Komponente Fasern (4) aufweist, welche dem Aufbau (2) zugeordnet werden können und die Träger für Kerne (3a, 4a) von Restenosis verhindernden Mitteln bilden, **dadurch gekennzeichnet, dass** die Kerne (3a, 4a) mindestens teilweise in Nanoteilchen (3) eingebaut sind, welche den Fasern (4) zugeordnet und mit einer Umhüllung (3b) aus bioerodierbarem Material versehen sind.

25. Komponente nach Anspruch 24, **dadurch gekennzeichnet, dass** die Kerne (3a, 4a) mindestens teilweise in den Fasern (4) enthalten sind, wobei die Fasern (4) aus bioerodierbarem Material bestehen.

26. Komponente nach Anspruch 25, **dadurch gekennzeichnet, dass** die Kerne (3a, 4a) mindestens teilweise in den Wänden der Fasern (4) eingebaut sind.

27. Komponente nach Anspruch 24, **dadurch gekennzeichnet, dass** die Fasern (4) hohle Fasern sind und dass die Nanoteilchen (3) innerhalb der Hohlräume der hohlen Fasern (4) angeordnet sind.

28. Komponente nach Anspruch 24 oder 27, **dadurch gekennzeichnet, dass** die Nanoteilchen (3) in verschiedenen Typen vorliegen, differenziert durch mindestens einen aus der nachfolgenden Gruppe ausgewählten Parameter:
- die Zusammensetzung des Kerns (3a),
- die Zusammensetzung der Umhüllung (3b),
- die Dicke der Umhüllung (3b),
- der relative Ort des Kerns (3a) in Bezug zur Umhüllung (3b),
- der Aufbau des Kerns (3a), und
- der Aufbau der Umhüllung (3b).

29. Komponente nach einem der Ansprüche 24 oder 27 bis 28, **dadurch gekennzeichnet, dass** die Nanoteilchen (3) Abmessungen im Wesentlichen zwischen 100 und 200 Nanometern aufweisen.

30. Komponente nach einem der vorhergehenden Ansprüche 24 bis 29, **dadurch gekennzeichnet, dass** die Fasern (4) in verschiedenen Typen vorliegen, differenziert durch mindestens einen aus der nachfolgenden Gruppe ausgewählten Parameter:
- die Zusammensetzung der Faser (4),
- die Dicke der Faser (4),
- der Aufbau der Faser (4), und
- der relative Ort des Kerns (3a, 4a) in Bezug zu der Faser (4).

31. Komponente nach einem der vorhergehenden Ansprüche 24 bis 30, **dadurch gekennzeichnet, dass** die Fasern (4) mindestens teilweise feste Fasern sind.

32. Komponente nach einem der vorhergehenden Ansprüche 24 bis 31, **dadurch gekennzeichnet, dass** die Fasern dem Aufbau (2) derart zugeordnet werden können, dass sie praktisch keinen Widerstand für die Aufweitung des Aufbaus von der radial zusammengezogenen Position zu der radial expandierten Position darstellen.

33. Komponente nach einem der vorhergehenden Ansprüche 24 bis 32, **dadurch gekennzeichnet, dass** die Fasern (4) mindestens teilweise mit dem Aufbau (2) verbunden sind.

34. Komponente nach einem der vorhergehenden Ansprüche 24 bis 33, **dadurch gekennzeichnet, dass** die Fasern (4) in Längsrichtung ausdehnbar sind.

35. Komponente nach Anspruch 32, **dadurch gekennzeichnet, dass** die Fasern (4) sich mindestens teilweise in der allgemeinen Richtung der Erzeugenden des im Wesentlichen rohrförmigen Aufbaus (2) erstrecken.

36. Komponente nach Anspruch 32, **dadurch gekennzeichnet, dass** die Fasern (4) mindestens teilweise in einem schraubenförmigen Weg mit einer Schraubensteigung von mindestens 45° gewickelt sind.

37. Komponente nach Anspruch 32, **dadurch gekennzeichnet, dass** die Fasern. (4) mindestens teilweise in einem schraubenförmigen Weg mit einer Schraubensteigung von mindestens 60° gewickelt sind.

38. Komponente nach einem der vorhergehenden Ansprüche 24 bis 37, **dadurch gekennzeichnet, dass** die Fasern (4) mindestens teilweise einen porösen Aufbau aufweisen.

39. Komponente nach einem der vorhergehenden Ansprüche 24 bis 38, **dadurch gekennzeichnet, dass** sie eine Einschließungshülle oder ein Häutchen (5) aufweist, das zwischen den Aufbau (2) und die Fasern (4) eingefügt werden kann.

40. Komponente nach Anspruch 39, **dadurch gekennzeichnet, dass** die Hülle (5) aus bioverträglichem Material besteht.

41. Komponente nach Anspruch 39 oder 40, **dadurch gekennzeichnet, dass** die Hülle (5) einen offenen Aufbau aufweist.

42. Komponente nach einem der Ansprüche 39 bis 41, **dadurch gekennzeichnet, dass** die Hülle (5) aus einem Silikonmaterial besteht.

43. Komponente nach einem der Ansprüche 39 bis 41, **dadurch gekennzeichnet, dass** die Hülle (5) aus einem metallischen oder polymeren Material besteht.

44. Komponente nach einem der vorhergehenden Ansprüche 39 bis 43, **dadurch gekennzeichnet, dass** die Hülle (5) mit einer Schicht aus bioverträglichem, Kohlenstoff enthaltendem Material beschichtet ist.

45. Komponente nach Anspruch 39, **dadurch gekennzeichnet, dass** die Fasern (4) mindestens teilweise in der Hülle (5) verankert sind.

46. Komponente nach einem der Ansprüche 24 bis 45, in Form eines kleinen Blattes, ausgebildet, um um den Aufbau des Stents (1) gewickelt zu werden.

47. Komponente nach einem der Ansprüche 24 bis 45, in Form einer rohrförmigen Socke, welche auf den Aufbau (2) des Stents (1) aufgebracht werden kann.

48. Sortiment von Komponenten gemäß einem der Ansprüche 24 bis 47, aufweisend eine Vielzahl von Komponenten, die **gekennzeichnet sind durch** die Anwesenheit von entsprechenden verschiedenen Typen des Restenosis verhindernden Mittels.

49. Sortiment von Komponenten gemäß einem der Ansprüche 24 bis 47, aufweisend eine Vielzahl von Komponenten, die **gekennzeichnet sind durch** entsprechende differenzierte Freigabekinetiken des Restenosis verhindernden Mittels.

## Revendications

1. Extenseur pour angioplastie comprenant une structure (2) dont l'enveloppe géométrique est essentiellement tubulaire et peut être dilatée depuis une position contractée radialement vers une position déployée radialement, dans lequel ladite structure (2) est pourvue de fibres (4) formant des vecteurs pour des noyaux (3a,4a) d'agents empêchant une nouvelle sténose, **caractérisé en ce que** lesdits noyaux (3a,4a) sont au moins partiellement (3a) incorporés dans des nanoparticules (3) associées auxdits fibres (4) et pourvus d'une enveloppe (3b) de matériau pouvant être bioérodé.

2. Extenseur selon la revendication 1, **caractérisé en ce que** lesdits noyaux (3a,4a) sont au moins partiellement contenus dans lesdites fibres (4), lesdites fibres (4) étant formées d'un matériau pouvant être bioérodé.

3. Extenseur selon la revendication 2, **caractérisé en ce que** lesdits noyaux (3a,4a) sont au moins partiellement (4a) incorporés dans les parois desdites fibres (4).

4. Extenseur selon la revendication 1, **caractérisé en ce que** lesdites fibres (4) sont des fibres creuses et lesdites nanoparticules (3) sont disposées à l'intérieur des cavités desdites fibres creuses (4).

5. Extenseur selon la revendication 1 ou la revendication 4, **caractérisé en ce que** lesdites nanoparticules (3) sont présentes selon différents types différentiés par au moins un paramètre choisi dans le groupe suivant :
- la composition du noyau (3a),
- la composition de l'enveloppe (3b),
- l'épaisseur de l'enveloppe (3b),
- l'emplacement relatif du noyau (3a) par rapport à l'enveloppe (3b),
- la structure du noyau (3a), et
- la structure de l'enveloppe (3b).

6. Extenseur selon l'une quelconque des revendications 1 ou 4 à 5, **caractérisé en ce que** lesdites nanoparticules (3) possèdent des dimensions approximatives comprises entre 100 et 200 nanomètres.

7. Extenseur selon l'une quelconque des revendications précédentes, **caractérisé en ce que** lesdites fibres (4) sont présentes selon différents types différenciés par au moins un paramètre choisi dans le groupe suivant :
- la composition de la fibre (4),
- l'épaisseur de la fibre (4),
- la structure de la fibre (4), et
- l'emplacement relatif desdits noyaux (3a,4a) par rapport à la fibre (4).

8. Extenseur selon l'une quelconque des revendications précédentes, **caractérisé en ce que** lesdites fibres (4) sont au moins en partie des fibres pleines.

9. Extenseur selon l'une quelconque des revendications précédentes, **caractérisé en ce que** lesdites fibres (4) sont fixées à ladite structure (2) de manière à ne présenter réellement aucune résistance à la dilatation de ladite structure depuis ladite position contractée radialement jusque dans ladite position déployée radialement.

10. Extenseur selon l'une quelconque des revendications précédentes, **caractérisé en ce que** lesdites fibres (4) sont interconnectées au moins partiellement à ladite structure (2).

11. Extenseur selon l'une quelconque des revendications précédentes, **caractérisé en ce que** lesdites fibres (4) sont extensibles longitudinalement.

12. Extenseur selon la revendication 9, **caractérisé en ce que** lesdites fibres (4) s'étendent, au moins en partie, dans la direction générale des génératrices de ladite structure essentiellement tubulaire (2).

13. Extenseur selon la revendication 9, **caractérisé en ce que** lesdites fibres (4) sont enroulées, au moins en partie, autour de ladite structure selon un trajet hélicoïdal avec un pas d'enroulement égal à au moins 45°.

14. Extenseur selon la revendication 9, **caractérisé en ce que** lesdites fibres (4) sont enroulées, au moins en partie, autour de ladite structure selon un trajet hélicoïdal avec un pas d'enroulement d'au moins 60°.

15. Extenseur selon l'une quelconque des revendications précédentes, **caractérisé en ce que** lesdites fibres (4) présentent, au moins en partie, une structure poreuse.

16. Extenseur selon l'une quelconque des revendications précédentes, **caractérisé en ce qu'**une gaine ou enveloppe de confinement (5) est intercalée entre ladite structure (2) et lesdites fibres (4).

17. Extenseur selon la revendication 16, **caractérisé en ce que** ladite gaine (5) est formée d'un matériau biocompatible.

18. Extenseur selon la revendication 16 ou la revendication 17, **caractérisé en ce que** ladite diode (5) possède une structure ajourée.

19. Extenseur selon l'une quelconque des revendications 16 à 18, **caractérisé en ce que** ladite diode (5) est réalisée en un matériau formé de silicone.

20. Extenseur selon l'une quelconque des revendications 16 à 18, **caractérisé en ce que** ladite diode (5) est formée d'un matériau métallique ou polymère.

21. Extenseur selon l'une quelconque des revendications précédentes 16 à 20, **caractérisé en ce que** ladite gaine (5) est recouverte par une couche d'un matériau biocompatible contenant du carbone.

22. Extenseur selon la revendication 16, **caractérisé en ce que** lesdites fibres (4) sont ancrées au moins partiellement dans ladite gaine (5).

23. Kit d'implantation comprenant :
- un extenseur selon l'une quelconque des revendications précédentes, et
- un cathéter (C) pour l'application in situ dudit extenseur (1).

24. Composant présentant une action empêchant une nouvelle sténose et qui est combiné à un extenseur pour angioplastie comprenant une structure (2) dont l'enveloppe géométrique est essentiellement tubulaire et qui peut être dilatée depuis une position contractée radialement vers une position déployée radialement, ledit composant comprend des fibres (4) qui peuvent être associées à ladite structure (2) et qui forment des vecteurs pour des noyaux (3a,4a) d'agents empêchant une nouvelle sténose, **caractérisé en ce que** lesdits noyaux (3a,4a) sont incorporés au moins partiellement (3a) dans des nanoparticules (3) associées auxdites fibres (4) et pourvues d'une enveloppe (3b) formée d'un matériau pouvant être bioérodé.

25. Composant selon la revendication 24, **caractérisé en ce que** lesdites fibres (4) sont des fibres creuses et que lesdites nanoparticules (3) sont situées à l'intérieur des cavités desdites fibres creuses (4).

26. Composant selon la revendication 25, **caractérisé en ce que** lesdits noyaux (39, 40) sont au moins partiellement (4a) incorporés dans les parois desdites fibres (4).

27. Composant selon la revendication 24, **caractérisé en ce que** lesdites fibres (4) sont des fibres creuses et lesdites nanoparticules (3) sont situés à l'intérieur des cavités desdites fibres creuses (4).

28. Composant selon la revendication 24 ou la revendication 27, **caractérisé en ce que** lesdites nanoparticules (3) sont présentes selon différents types différenciés par au moins un paramètre choisi dans le groupe suivant :
- la composition du noyau (3a),
- la composition de l'enveloppe (3b),
- l'épaisseur de l'enveloppe (3b),
- l'emplacement relatif du noyau (3a) par rapport à l'enveloppe (3b),
- la structure du noyau (3a), et
- la structure de l'enveloppe (3b).

29. Composant selon l'une quelconque des revendications 24 ou 27 à 28, **caractérisé en ce que** lesdites nanoparticules (3) ont des dimensions situées essentiellement entre 100 et 200 nanomètres.

30. Composant selon l'une quelconque des revendications précédentes 24 à 29, **caractérisé en ce que** lesdites fibres (4) sont présentes selon différents types différenciés par au moins un paramètre choisi dans le groupe suivant :
- la composition de la fibre (4),
- l'épaisseur de la fibre (4),
- la structure de la fibre (4), et
- l'emplacement relatif desdits noyaux (3a,4a) par rapport à la fibre (4).

31. Composant selon l'une des revendications précédentes 24 à 30, **caractérisé en ce que** lesdites fibres (4) sont au moins en partie des fibres pleines.

32. Composant selon l'une quelconque des revendications précédentes 24 à 31, **caractérisé en ce que** lesdites fibres (4) peuvent être associées à ladite structure (2) de manière à ne présenter réellement aucune résistance à la dilation de ladite structure depuis ladite position contractée radialement jusqu'à ladite position déployée radialement.

33. Composant selon l'une quelconque des revendications précédentes 24 à 32, **caractérisé en ce que** lesdites fibres (4) peuvent être au moins partiellement interconnectées à ladite structure (2).

34. Composant selon l'une quelconque des revendications précédentes 24 à 33, **caractérisé en ce que** lesdites fibres (4) sont extensibles longitudinalement.

35. Composant selon la revendication 32, **caractérisé en ce que** lesdites fibres (4) s'étendent, au moins en partie, dans la direction générale des génératrices de ladite structure essentiellement tubulaire (2).

36. Composant selon la revendication 32, **caractérisé en ce que** lesdites fibres (4) sont enroulées, au moins en partie, autour de ladite structure selon un trajet hélicoïdal avec un pas d'enroulement égal à au moins 45°.

37. Composant selon la revendication 32, **caractérisé en ce que** lesdites fibres (4) sont enroulées, au moins en partie, autour de ladite structure selon un trajet hélicoïdal avec un pas d'enroulement d'au moins 60°.

38. Composant selon l'une quelconque des revendications 24 à 37, **caractérisé en ce que** lesdites fibres (4) présentent, au moins en partie, une structure poreuse.

39. Composant selon l'une quelconque des revendications 24 à 38, **caractérisé en ce qu'**il comprend une gaine de confinement (5) est intercalée entre ladite structure (2) et lesdites fibres (4).

40. Composant selon la revendication 39, **caractérisé en ce que** ladite gaine (5) est formée d'un matériau biocompatible.

41. Composant selon la revendication 39 et la revendication 40, **caractérisé en ce que** ladite gaine (5) possède une structure ouverte.

42. Composant selon l'une quelconque des revendications 39 à 41, **caractérisé en ce que** ladite gaine (5) est formée d'un matériau silicone.

43. Composant selon l'une quelconque des revendications 39 à 41, **caractérisé en ce que** ladite gaine (5) est formée d'un matériau métallique ou polymère.

44. Composant selon l'une quelconque des revendications précédentes 39 à 43, **caractérisé en ce que** ladite gaine (5) est recouverte d'une couche d'un matériau biocompatible contenant du carbone.

45. Composant selon la revendication 39, **caractérisé en ce que** lesdites fibres (4) sont au moins partiellement ancrées sur ladite gaine (5).

46. Composant selon l'une quelconque des revendications 24 à 45, sous la forme d'une petite feuille conçue pour être enroulée autour de la structure de l'extenseur (1).

47. Composant selon l'une quelconque des revendications 24 à 45, sous la forme d'un manchon tubulaire apte à être placé sur la structure (2) de l'extenseur (1).

48. Assortiment de composants selon l'une quelconque des revendications 24 à 47, comprenant une pluralité desdits composants, qui sont **caractérisés par** la présence de types respectifs différents dudit agent empêchant une nouvelle sténose.

49. Assortiment de composants selon l'une quelconque des revendications 24 à 47, comprenant une pluralité desdits composants qui sont **caractérisés par** des cinétiques différenciées respectives de libération dudit agent empêchant une nouvelle sténose.
